# EUROPEAN PATENT APPLICATION

(11) **EP 0 556 878 A2**
(43) Date of publication of application: **25.08.1993**
(21) Application number: 93107864.6
(22) Date of filing: 14.10.1988
(51) Int. Cl.: A61M 5/145, F16H 21/20

(54) **Thermoelectric chiller and automatic syringe**

(62) Divisional of application: 88117082.3
(71) Applicant: Becton Dickinson and Company, Franklin Lakes, New Jersey 07417-1880 (US)
(72) Inventor: Streeter, James M., Highland, Utah (US)
(74) Representative: Selting, Günther, Dipl.-Ing.

(57) **Abstract**

An automatic syringe drive assembly (10) and disposable heat exchanger cassette (35) are used in combination to provide an improved thermodilution technique and apparatus. The automatic syringe drive assembly includes replaceable connecting rods (20) on a slider (19) crank (21) mechanism which permit easy and accurate adjustment of the syringe injection volume. The drive assembly supports an insulated syringe (11) for use in making the injection.

## Description

The invention relates to a mechanism for driving a plunger of a syringe to inject a predetermined amount of injectate bolus volume, in particular for supplying an exactly measured bolus of a sterile injectate into a thermodillution catheter.

From US-A-2 164 711, an instrument for drawing blood is known, wherein the instrument body has a cavity serving as a syringe in which a plunger is linearly movable. The plunger is driven by a crank which can be rotated around its center axis. The crank is connected to a connecting rod for converting the rotary movement of the crank into a linear movement of the plunger. The crank may be rotated by the hand of the operator.

Motor actuated syringes have been subject of numerous prior art patents, for example, U.S. Patent Nos. 3,336,925; 3,156,236; 3,335,724; 2,896,621; 2.457,977; 3,313,291; 2,602,446; 2,498,672; and 3,584,623. However, none of these devices include an easily adjustable mechanism for accurately varying the amount of volume injected.

It is an object of the present invention to provide a mechanism for driving a plunger of a syringe, which allows for accurately varying the amount of the liquid volume to be injected.

This object is solved, according to the invention, with the features of claim 1 or 3, respectively.

More specifically, the disposable portions of the system are designed to be inexpensive and effective and easily used in combination with the basic mechanism. The basic system disclosed has an automatic syringe driving mechanism which by merely changing a connecting rod will provide various stroke volumes without concern for sophisticated levels of adjustment.

The automatic injectate syringe driving system includes a gear-reduced motor in combination with a slider crank mechanism, the connecting rod for which is easily interchanged to increase or decrease stroke and, therefore, the injectate volume. In the preferred embodiment various connecting rod lengths are provided and are selected to drive the syringe plunger in accordance with the stroke volume desired.

Figure 1 is a plan view of the automatic injection drive mechanism composed of a slider crank mechanism for the preferred embodiment.

Figure 2 is a perspective view partially cut away of the disposable heat exchanger cassette of the preferred embodiment.

Figure 3 is a top plan view with the housing in section showing the organization of the disposable heat exchanger cassette and the thermoelectric device used to change the temperature of the injectate bolus.

Figure 4 is a block diagram showing the relationship of the components in the automatic injectate system of the present invention.

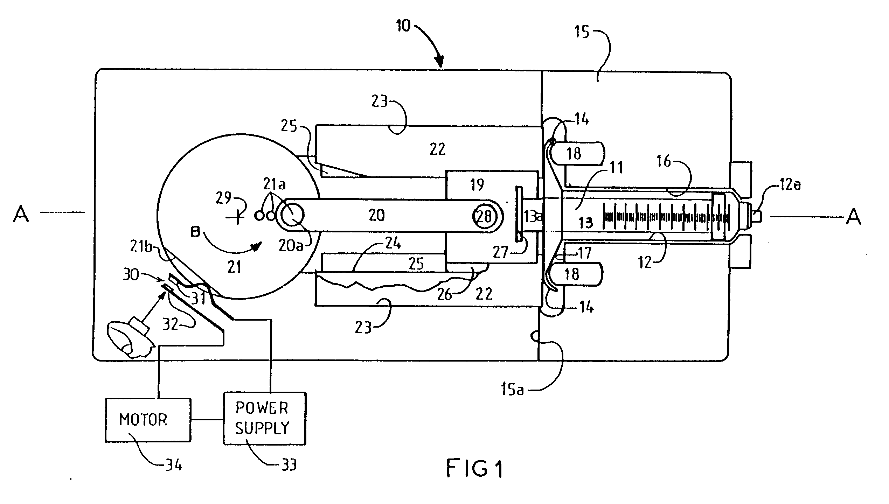

In figure 1 is an automatic syringe drive assembly 10 which supports an insulated syringe 11. The syringe 11 has a doubled wall insulated syringe barrel 12 and a plunger 13 which slides within the inner wall of barrel 12 of the syringe 11 to force fluids through the luer connector 12a located at the distal end of the insulated syringe 11. In the context of this description distal means toward the luer connection 12a and proximal means in the opposite direction. At the proximal end of the insulated syringe 11 are a pair of extending finger handles 14 which in normal or manual use are designed to fit the fingers of the syringe user. In the application of the automatic syringe drive assembly 10 the syringe 11 is held in a chassis 15 which is a heavy metal plate having specifically shaped openings to carry the components of the automatic syringe drive assembly 10. In particular, there is a syringe barrel recess 16 adapted and configured to support the insulated barrel 12 against side to side or axial movement.

At the proximal end of recess 16 in chassis 15 is a transverse recess 17 which extends outwardly from recess 16 to form a "T" shaped opening and to receive the handles 14. Adjustable tabs 18 are positioned parallel to recess 16 on each side thereof but spaced therefrom and each tab 18 extends into a part of handle recess 17 to bear against and hold the finger fitting portions of the handles 14. Adjustable tabs 18 can be moved toward and away from the handles 14 to cause each handle 18 to bear against its more proximal side of the recess 17.

In order to drive the plunger 13 there is a flat slider 19 which is driven by a connecting rod 20 rotated by a crank 21 at the connecting rod proximal end. The slider crank mechanism composed of the connecting rod 20, the crank 21 and the slider 19 convert the rotary motion of the crank 21 to reciprocating motion of the slider 19. The reciprocatory motion of slider 19 is controlled by guides 22 positioned within the chassis 15.

Specifically, the guides 22 have a groove arrangement for controlling the motion of a tongue on each side of slider 19. The particular components of the tongue and groove consist of chassis mounting channels 23 located parallel to slider 19 and carried within chassis 15. The guides 22 are received and supported in the operating plane of the insulated syringe 11, the slider 19, and the crank 21 whereby reciprocating motion of the slider 19 is in line with axis A-A of the plunger 13, see figure 1. Mounting channels 23 hold the guides 22 in position for supporting the slider 19. There is a groove 24 in each of the guides 22 which control the slider 19 for sliding reciprocatory motion in line with the axis A-A of plunger 13. Each groove 24 is formed within its respective guide 22 by a pair of extending flanges 25 designed to carry therebetween a tongue 26 extending transverse to and outwardly from each of the sides of piston 19. Consequently, as slider 19 reciprocates in response to forces transmitted through connecting rod 20 a tongue 26 on each side of slider 19 move within its respective groove 24. Motion of slider 19 is thus kept within the plane and axially relative to plunger 13.

In the distal end of slider 19 is a "T" shaped opening 27 which receives the end 13a of the plunger 13 to permit movement of the plunger 13 in the distal and proximal directions due to reciprocation of slider 19. At the proximal end of slider 19 is pin 28 which operates in a conventional fashion to allow pivotal motion between the distal end of the connecting rod 20 and the slider 19.

Crank 21 is supported by a shaft in a known manner for rotation about the crank center 29 in the direction shown by arrow B in figure 1. Chassis 15 is stepped at wall 15a to provide clearance for moving parts, 19, 20 and 21. The motor is shown schematically in figure 1 but is actually located beneath the chassis 15 to drive crank 21 about its center 29. Crank 21 has a series of threaded holes 21a positioned along a radius which are used in cooperation with connecting rods of varying length. Longer connecting rods 20 in combination with the arrangement shown in figure 1 will provide a shorter plunger stroke. A replaceable pin 20a connects the proximal end of connecting rod 20 to the crank 21. Pin 20a is in the nature of a shouldered threaded fastener that can easily be unscrewed from the hole 21a allowing replacement of the connecting rod 20 with another rod having a different length. The distal end of connecting rod 20 is designed to slip over the pin 28. Since the motion of slider 19 is kept in the plane of reciprocation by the tongue and groove arrangement, no unseating forces are incurred at the connection of pin 28.

Crank 21 also includes a cam flat 21b which is used to control the starting and stopping point for the syringe drive assembly 10. Shown schematically is a switch 30 having a cam movable contact 31 which is responsive to cam flat 21b and a manually movable contact 32 such that switch 30 is normally closed except when movable contact 31 is against cam flat 21b. When against the cam flat 21b the switch 30 is opened and the circuit for the automatic syringe drive assembly 10 is disconnected. The manually operated contact 32 can be used to close switch 30 and start the automatic syringe drive 10. The operating circuit includes a power supply 33, one leg of which is connected to contact 32 and the other leg is connected to a motor 34 (shown schematically in figure 1). The other input for motor 34 is connected to the movable contact 31. When the crank 21 is in the position of figure 1 and the cam flat 21b has permitted moving contact 31 to break its connection from manually operated contact 32 and the motor 34 is disconnected from the power supply 33. Consequently, switch 30 can be overridden to activate the automatic syringe drive assembly 10. Switch 30 is designed to control the position at which the slider 19 begins and ends its reciprocatory motion. With this arrangement the mechanism of the automatic syringe drive assembly 10 can be manually started and automatically stopped for use in providing one complete stroke. The plunger 13 begins and ends each stroke at the same point, i.e. the most distal end of its travel with the particular length of connecting rod used. The stroke of plunger 13 is easily and accurately changed. The volume of injection is entirely automatic and easily and accurately set.

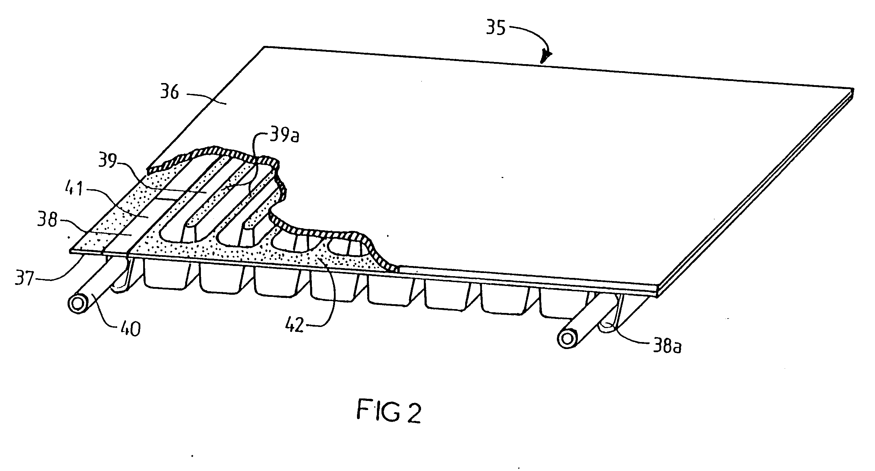

Figure 2 shows the perspective view of a heat exchanger cassette 35 used in connection with the automatic syringe drive assembly 10. The heat exchanger cassette 35 consists of a conductive plate 36 formed of stainless steel sheet being 0.25 mm (.010 inches) thick and measuring approximately 6.7 cm (2.75 inches) by 10.7 cm (4.375 inches). These dimensions can be varied for the particular application and the stainless steel is a suitable grade and finish for medical use. Plate 36 is adhesively connected to a thermoformed plastic labyrinth 37 which has repetitive S-shaped convolutions. At the ends of the labyrinth 37 are specifically shaped connectors 38 arranged to terminate the open ends of the labyrinth 37. The labyrinth 37 consists of a channel 39 which is U-shaped in cross-section and forms a circuitous path by extending back and forth across beneath the plate 36 to form parallel passageways having a hair pin turn at each end. The open ends of channel 39 are filled with connectors 38 which convert the generally U-shaped channel cross-section to a circular cross-section extending male tubing extension 40. A U-shaped portion 38a on each connector 38 is adhesively sealed into an open end of channel 39 with an extended fitting portion 41. Through extended fitting portion 41 passes a passage (not specifically shown) carefully shaped to gently funnel the flow from the U-shaped channel 39 to the circular cross-section of the male luer tubing extension 40.

Between each U-shaped channel 39 there are flat pad-like areas 39a positioned to rest against one of the major surfaces of the conductive plate 36. Between the areas 39a and the plate 36 is placed hot melt adhesive 42 which seals the labyrinth 37 to the plate 36 forming a fluid tight circuitous conductive path through the U-shaped channel 39. In particular, at the ends of channel 39 are connectors 38 which permit fluid to flow from one side of the heat exchanger assembly 35 to the other. As will be explained in detail, fluid passing through the labyrinth 37 formed by channel 39 exchanges heat. The volume of fluid contained by the preferred heat exchanger 35 is at least three times the stroke volume of the automatic syringe drive 10. Heat exchanger 35 is effective because the conductive plate 36 is metal and the labyrinth 37 is plastic. The combination is easily constructed and efficient in operation. It is also low cost, and, therefore, disposable. An additional safety feature is the hot melt adhesive 42 which cannot be autoclaved. Sterilization must be by radiation or ethylene oxide gas.

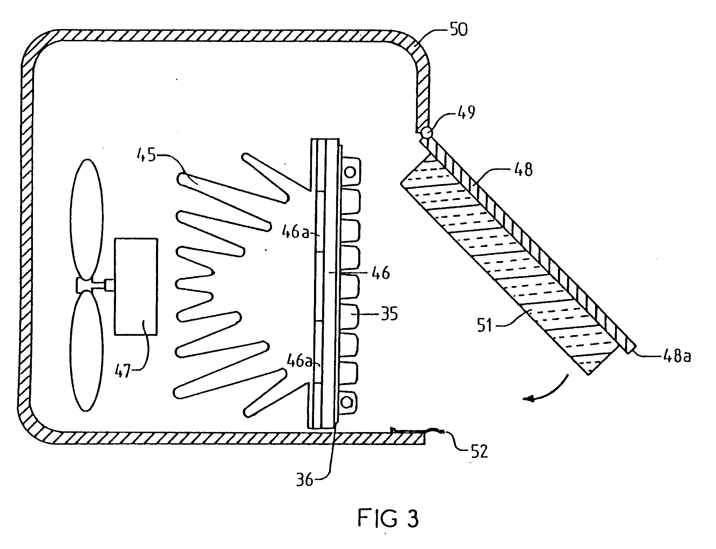

In operation the conductive plate 36 is held against the thermoelectric surface of a chiller whereby fluid passing through the labyrinth 37 is lowered in temperature to that of the conductive plate 36, see figure 3. In a preferred arrangement a thermoelectric chiller assembly 44, having a finned heat exchanger 45 with a series of junctions of dissimilar metals, causes the temperature of the finned heat exchanger 45 to change when a current is passed through the junctions. Figure 3 is a top plan view of the arrangement for the thermoelectric chiller 44. More specifically, the way in which the disposable heat exchanger cassette 35 is brought to bear against the chilling surface of the thermoelectric chiller 44 is shown. The finned heat exchanger 45 is manufactured from an aluminum extrusion and is designed to support a chilled surface 46 which included the thermoelectric devices 46a that receive electrical current. Chilled surface 46 can include a thin coating of electrical insulation such as ceramic for safety. Depending upon the direction of electric current flow through devices 46a, the temperature will increase or decrease. In the preferred application temperature decrease is used to chill the surface 46. Opposite the cold surfaces of devices 46a are hot surfaces connected to finned heat exchangers 45 for heat removal. A fan assembly 47 is provided to move air across the finned heat exchanger 45 and thus lower the temperature of the heat exchanger 45 to ambient.

A door 48 carried upon a hinge 49 is supported upon a housing 50 for heat exchanger 45. The combination of door 48, hinge 49 and housing 50 permits easy replacement of the disposable heat exchanger cassette 35. The door 48 carries a resilient insulating foam pad 51 which is designed to bear against the labyrinth 37 of the heat exchanger cassette 35 and hold the conductive plate 36 against the chilled surface 46. It should be appreciated that the heat exchanger assembly 35 is in the nature of a disposable medical device so that with each procedure a new cassette can be used without complications or difficulty. This is important to prevent transmission of infection. The connectors 38 have male tubing extensions 40 which easily attach to the administration set on one end and the automatic syringe drive assembly 10 (at the luer connection 12a) on the other end. For that purpose there is a special tubing set which fits over extensions 40 and adapts to luer connections.

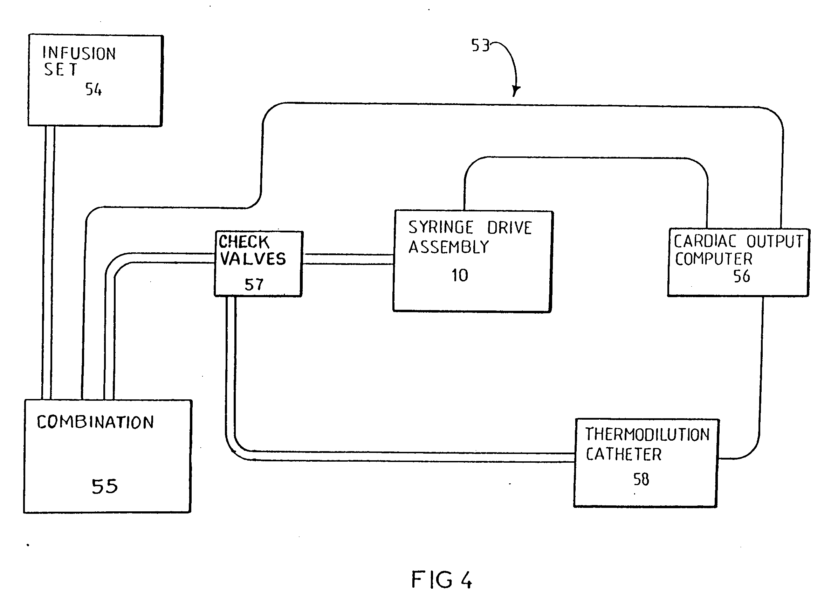

In order to hold the insulating foam pad 51 against the heat exchanger cassette 35, there is a housing latch 52 which cooperates with the edge of the door 48. In operation the chiller assembly 44 in combination with the cassette 35 (as shown in figure 3) function to replace the normal coil of flexible tubing and ice bath used in the American an Edwards Coset™ apparatus. That is to say that the arrangement schematically shown in figure 4 is known to medical practitioners except for the automatic syringe drive 10, the chiller 44 and disposable cassette 35 which are herein shown and describe. The coil and ice bath are replaced with the apparatus of figure 3. All of the plumbing including valves and connections are identical to that with which medical practitioners are familiar and that plumbing is arranged and used in accordance with conventional techniques. The apparatus of the present disclosure provides automatic repetitive and adjustable chilling of a bolus and is accurate, convenient and easy to use. In operation, the device of the present invention provides an accurately measured bolus at a preferred temperature, both of which are readily and automatically controlled in volume and temperature.

Figure 4 is a block diagram of the automatic injection system 53 for purposes of explaining how the automatic syringe drive assembly 10, the disposable heat exchanger 35 and the thermoelectric chiller assembly 44 are used. The injectate is drawn from an infusion set 54 into the combination 55 which includes the thermoelectric chiller assembly 44 and disposable heat exchanger cassette 35. The operation of the syringe drive assembly 10 can be automatically controlled by a cardiac output computer 56 which is electrically connected to the automatic syringe drive assembly 10 and the combination 55 of disposable heat exchanger 35 and thermoelectric chiller assembly 44. Once the temperature of the injectate drawn from the infusion set 54 has reached the proper temperature, a signal is transmitted from the combination 55 to the computer 56 and in response thereto the automatic syringe drive assembly 10 is activated by means of its electrical connection to computer 56.

The flow of injectate as pumped by the syringe drive assembly 10 is controlled by check valves 57. The check valves 57 are arranged to permit fluid to first flow from the infusion set 54 to fill the combination 55 and then from there to thermodilution catheter 58. More specifically, the syringe drive assembly 10 is first filled from the infusion set 54 and then the syringe plunger 13 forces the injectate back through check valves 57 and into the thermodilution catheter 58. The check valves 57 are merely two one-way valves which permit flow from the infusion set 54 through the combination 55 (filling disposable heat exchanger cassette 35 and syringe) and then from the automatic syringe 10 and combination 55 to the thermodilution catheter 58.

The thermodilution catheter 58 is placed within the patient. In the patient end of the thermodilution catheter 58 is a thermistor which is electrically connected to the cardiac output computer 56 such that the temperature of that portion of the catheter can be monitored by the cardiac output computer.

To operate the combination 55 is electrically activated and filled with injectate by cycling syringe drive assembly 10. The automatic syringe drive assembly 10 is run through one cycle such that a volume of injectate is drawn from the infusion set 54 and purged through the system. During the further the ambient injectate within the system is delivered to the patient. The volume of the disposable heat exchanger cassette 35 is at least three times that of one stroke of the syringe plunger 13. Therefore, the purge cycle is adequate to deliver all of the ambient injectate into the patient and leave the system completely filled with chilled injectate.

The system can be operated entirely automatically because of the electrical connection between the combination 55 and the cardiac output computer 56, as well as the electrical connection between the automatic syringe drive assembly 10 and the cardiac output computer 56. After the cardiac output computer 56 receives a signal from the thermistor of the thermodilution catheter 58 (i.e. the temperature of the patient end of the thermodilution catheter 58 has stabilized) the signal to inject a first chilled bolus is given by the cardiac output computer 56 to the automatic syringe drive 10.

As described in connection with figure 1, there is a manual override for switch 30 to activate the syringe drive 10. For fully automatic operation a shunt between the switched connections from the power supply 33 and the motor 34 will begin the operation of the automatic syringe 10. This shunt can be provided by the cardiac output computer 56 for a sufficient time to run the motor past the cam flat 21b and switch 30 will then complete the circuit and run the motor 34 for one complete cycle of the plunger 13.

Automatic plunger operation is qualified by the temperature of the chilled injectate which is measured in the chilled surface 46 of the thermoelectric chiller assembly. When that chilled surface 46 is at its operating temperature, the cardiac output computer 56 will start the cycle of the syringe drive assembly 10. The injection will be completed at the point where the movable contact 31 of switch 30 has again reached the cam flat 21b. The system is capable of manual operation. The thermoelectric chiller and cassette having reached operating temperature, a medical practitioner can manually start the cardiac output computer 56, and then the automatic syringe drive 10. The system when automatically or manually operated provides accurate and repeatable volumes of carefully cooled injectate.

## Claims

1. A mechanism for driving a plunger (13) of a syringe (14) to inject a predetermined amount of injectate bolus volume, comprising a rotary drive crank (21) and a connecting rod means (20) disposed between said crank (21) and the syringe plunger (13), for converting the rotary motion of the crank into a linear movement of the syringe plunger (13),
**characterized in that**
the syringe (11) is removable supported in a retainer portion (16) of a support (15), said support carrying the crank (21) and having a linear guide including a pair of slides (22) forming a guideway,
a slider (19) for the end of the plunger (13) is provided, said slider being shaped to fit between the linear guide slides (22) for defining a reciprocating motion in alignment with the syringe plunger (13), the slider (19) being connected to a first end of said connecting rod means (20),
and a second end of the connecting rod means (20) being coupled to said crank (11) so that it is capable of being positioned in various locations relative to the crank (21) for changing the distance of linear motion of the slider (19).

2. The mechanism of claim 1 wherein said connecting rod means (20) comprises various length connecting rods for different stroke lengths of the syringe plunger, resulting in preselected bolus volumes of injectate according to the connecting rod used, and wherein said rods are connected to said crank and said holder so that said rods of various lengths can be easily exchanged to adjust bolus volume.

3. A mechanism for driving a plunger (13) of a syringe (11) to inject a predetermined amount of injectate bolus volume, comprising
a first means (15) for supporting an insulated syringe barrel (12) aligned with an axis,
a second means associated with said first means (15) for guiding a slider (16) driving the plunger (13) of the syringe (11) in alignment with said axis of said barrel,
a third means (21) mounted to said first means (15), for providing rotary motion in the plane of said axis and carried on a rotary center (29) normal to said axis,
a fourth means (20) connected between said third means (21) and said slider (16) for converting the rotary motion of said third means (21) into reciprocatory motion at said slider (16), thus driving the plunger (13),
a fifth means (20a) variably associated with said third means (21), for varying the length of said fourth means (20) whereby the stroke of the plunger (13) can be adjusted to predetermined values to obtain preselected volumes of bolus injectate.

4. The injection system of claim 3, wherein said fifth means (20a) is also associated with said fourth means (20) which includes a selection of connecting rods of varying lengths each designed to provide a given stroke length of the plunger (13) and each adapted to extend from a different area of said third means (21) to said second means (20) in a predetermined way for easy substitution.

5. The injection system of claim 4, wherein said extension from said different area is in a predetermined way for easy substitution and includes a crank arm length defined by a threaded journal (21a) located on said third means (21) at different distances along a radius thereof and relative to the rotary center (29) of said third means (21).

6. The system of anyone of claims 1-5, wherein said third means or crank (21) cooperates with a switched motor (34) to start and cycle the plunger (13) one revolution of said third means.
